(19) Europäisches Patentamt
European Patent Office
Office européen des brevets

(11) Veröffentlichungsnummer: **0 004 322**
**B1**

(12) # EUROPÄISCHE PATENTSCHRIFT

(21) Anmeldenummer: **79100712.3**

(22) Anmeldetag: **09.03.79**

(51) Int. Cl.[3]: **C 07 D 217/22,**
C 07 D 403/04,
C 07 D 409/04,
A 61 K 31/47

(54) **Isochinolinderivate, Verfahren zu ihrer Herstellung und ihre Verwendung zur Herstellung von Arzneimitteln**

(30) Priorität: **16.03.78 DE 2811361**

(43) Veröffentlichungstag der Anmeldung:
**03.10.79 Patentblatt 79/20**

(45) Bekanntmachung des Hinweises auf die Patenterteilung:
**29.10.80 Patentblatt 80/22**

(84) Benannte Vertragsstaaten:
**CH DE FR GB**

(56) Entgegenhaltungen:
**Keine**

(73) Patentinhaber: **HOECHST AKTIENGESELLSCHAFT**
**Zentrale Patentabteilung Postfach 80 03 20**
**D - 6230 Frankfurt Main 80 (DE)**

(72) Erfinder: **Bartmann, Wilhelm, Dr.**
**Am Dauchsbau 5**
**D - 6232 Bad Soden am Taunus (DE)**
**Konz, Elmar, Dr.**
**Buchenweg 22**
**D - 6232 Bad Soden am Taunus (DE)**
**Kruse, Hansjörg, Dr.**
**Feldbergstrasse 70**
**D - 6233 Kelkheim (Taunus) (DE)**

EP 0 004 322 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Isochinolinderivate, Verfahren zu ihrer Herstellung und ihre Verwendung zur Herstellung von Arzneimitteln

Die Erfindung betrifft substituierte Isochinolin-4-aldehyde mit wertvollen pharmakologischen, insbesondere psychotropen Eigenschaften.

Gegenstand der Erfindung sind Isochinolin-4-aldehyde der allgemeinen Formel I,

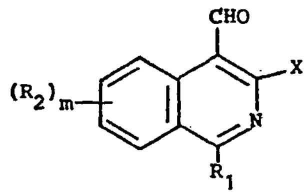

I

worin

X Brom oder Chlor bedeutet,

$R_1$ einen Phenylrest, der gegebenenfalls mit Halogen-, Hydroxy-, Nitro-, Amino- oder einer durch ein oder zwei aliphatische oder cycloaliphatische Kohlenwasserstoffreste substituierten Aminogruppe mit zwei bis achtzehn Kohlenstoffatomen, einer Alkyl- oder ALkoxygruppe mit jeweils einem bis sechs Kohlenstoffatomen, einer Benzyloxy- oder einer Trifluormethyl-gruppe mono- oder disubstituiert ist, einen Pyridyl- oder Thienylrest bedeutet,

$R_2$ Wasserstoff, Halogen- Hydroxy-, Alkyl- oder Alkoxygruppe mit einem bis sechs Kohlenstoffatomen, Nitro-, Amino-, Benzyloxy, Methylendioxy- oder Äthylendioxygruppe darstellt und

m eins oder zwei darstellt,

deren physiologisch verträglichen Salze, Verfahren zu ihrer Herstellung, pharmakologische Zubereitungen und ihre Verwendung.

Insbesondere beinhaltet die Erfindung Verbindungen, worin X gleich Chlor ist, $R_1$ einen Pyridyl- oder Phenylring darstellt, letzterer mit Hydroxy-, Halogen-, Nitro-, Amino- oder einer mit zwei gleichen aliphatischen Kohlenstoffresten mit jeweils einem bis vier Kohlenstoffatomen substituierten Aminogruppe, einer Alkyl- oder Alkoxygruppe mit jeweils einem bis vier Kohlenstoffatomen mono- oder disubstituiert ist, m gleich 1 oder 2 ist und $R_2$ Wasserstoff, Halogen, eine Alkyl- oder Alkoxygruppe mit einem bis vier Kohlenstoffatomen oder Hydroxygruppe darstellt.

Ganz besonders beinhaltet die Erfindung Verbindungen, worin X gleich Chlor ist, $R_1$ einen Phenylring darstellt, der mit Halogen, Hydroxy, Nitro, $C_1$—$C_3$-Alkyl-, Amino- oder Methoxygruppe mono- oder disubstituiert ist, m gleich 1 oder 2 und $R_2$ Wasserstoff, Halogen, Hydroxy, $C_1$—$C_3$-Alkyl- oder Methoxygruppe darstellt.

Das Verfahren zur Herstellung der Verbindung der Formal I ist dadurch gekennzeichnet, daß man

a) Verbindungen der Formel II

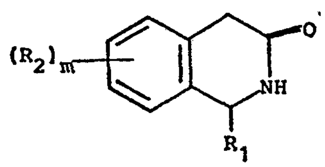

II

worin $R_1$, $R_2$ und m die zur Formel I genannte Bedeutung haben, mit einem Vilsmeier-Addukt aus einem Säureamid wie z.B. Dimethylformamid, Diäthylformamid, N,N-Methylphenylformamid oder N-Formylpiperidin und einem Säurechlorid wie z.B. Phosphoroxichlorid, Thionylchlorid, Phosgen oder Säurebromid wie Phosphoroxibromid, Phosphortribromid zu Verbindungen der Formell III umsetzt,

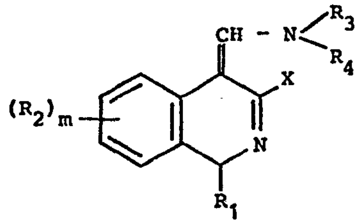

III

worin X gleich Chlor oder Brom ist und $R_3$ und $R_4$ Alkyl oder Cycloalkyl mit einem bis sechs Kohlenstoffatomen oder Phenyl bedeutet, und diese anschließend zu Verbindungen der Formel I oxydiert, oder

b) eine Verbindung der Formel IV

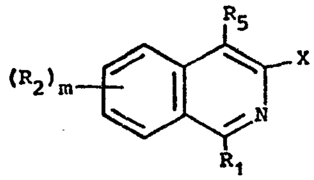

IV

worin $R_5$ einen Methyl-, Hydroxymethyl oder Aminomethylrest bedeutet, der am Stickstoffatom durch einen Alkylrest mit 1 bis 4 Kohlenstoffatomen substituiert sein kann und X, $R_1$, $R_2$ und m die oben genannte Bedeutung haben, zu einer Verbindung der Formel I oxydiert, oder

c) eine Verbindung der Formel V

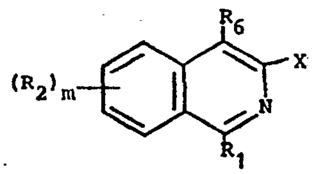

V

worin $R_6$ eine Carboxylgruppe oder ein Carbonsäurederivat bedeutet und X, $R_1$, $R_2$ und m die oben genannte Bedeutung haben, zu einer Verbindung der Formel I reduziert, oder

d) eine Verbindung der Formel VI

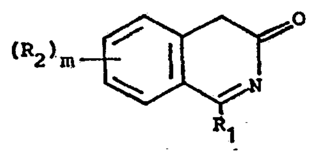

VI

worin $R_1$, $R_2$ und m die oben genannte Bedeutung haben, mit dem Vilsmeier-Haack Komplex zu einer Verbindung der Formel I umsetzt, oder

e) eine Verbindung der Formel VII

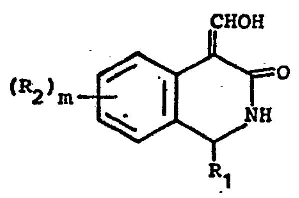

VII

worin $R_1$, $R_2$ und m die oben gennante Bedeutung haben, mit einem Säurechlorid, z.B. $POCl_3$, $SOCl_2$, zu einer Verbindung der Formel I umsetzt, oder

f) eine Verbindung der Formel I

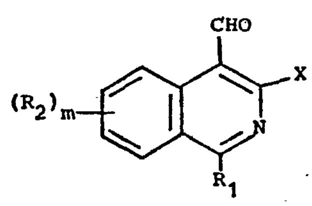

I

worin X, $R_2$ und m die zur Formel I genannte Bedeutung haben und sofern $R_1$ den Phenylring darstellt, diesen Phenylring nachträglich substituiert, oder

g) einen Rest $R_2$ oder gegebenenfalls am Phenylring in 1-Stellung vorhandene Substituenten so verändert, daß weitere Verbindungen der allgemeinen Formel I entstehen.

Di Darstellung der Ausgangsverbindungen der allgemeinen Formel II für das *Verfahren a)* ist in

der DE—OS 22 25 669 und in Acad.Sci.Hung *60* (1969), Seite 177 beschrieben. Dabei geht man so vor, daß man ein Nitril oder ein Amid der Formel

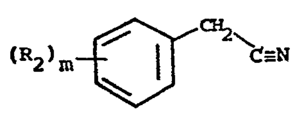 oder 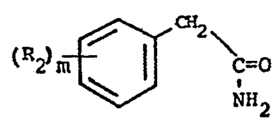

mit einem Aldehyd der Formel $R_1-\overset{H}{\overset{|}{C}}=O$ in Phosphorsäure, deren Gehalt an Phosphorpentoxid zwischen dem von 85 %iger Phosphorsäure und dem von Polyphosphorsäure variieren kann, zur Reaktion bringt. Man arbeitet dabei bei Temperaturen von Raumtemperatur bis 180°C, wobei eine Temperatur von 80° bis 110°C bevorzugt wird. Die weitere Umsetzung nach Vilsmeier-Haack wird in an sich bekannter Weise vorgenommen (O.Bayer in Houben-Weyl: Methoden der organischen Chemie, 4.Auflage Y.Thieme, Stuttgart 1954, Bd. 7/1, Seite 29). Dabei geht man so vor, daß man Verbindungen der allgemeinen Formel II mit z.B. Dimethylformamid/Phosphoroxichlorid bzw. Phosphoroxibromid zwischen 0°C und 30°C umsetzt. Als Lösungmittel kommen indifferente, wasserfreie organische Lösungsmittel, wie Chloroform, Tetrachlorkohlenstoff, Tetrahydrofuran, Dioxan, Benzol, Toluol, Chlorbenzol, N,N-Dimethylformamid usw. in Frage. Der Vilsmeier-Komplex wird mindestens in äquivalenten Mengen, vorzugsweise mit 3—5fachen Überschuß angewendet. Die Oxydation der Verbindung III kann in üblicher Weise vorgenommen werden. Dabei setzt man als Oxydationsmittel Kaliumpermanganat, Chromsäure oder Mangandioxyd ein und arbeitet in wässriger Lösung, oder in heterogenen Systemen, wobei die wässrige Phase einen neutralen bis sauren pH-Wert besitzt, vorzugsweise pH 1 bis 6. Bei der Arbeitsweise im heterogenen System kommen als Lösungsmittel mit Wasser nicht mischbare, indifferente aprotische Lösungsmittel wie z.B. Benzol, Toluol, Chlorbenzol, Chloroform oder Tetrachlorkohlenstoff in Frage.

Gemäß *Verfahrensvariante b)* werden die erfindungsgemäßen Verbindungen durch Umsetzung einer Verbindung der Formel IV mit oxydierenden Mitteln z.B. Mangandioxyd erhalten. Derartige Oxydationsreaktionen sind prinzipiell bekannt. (vgl. z.B. "Compendium of Organic Synthetic Methods", Verlag John Wiley a. Sons, Inc. (1971), Seiten 146 bis 147, 150 bis 152).

Auch die *Verfahrensvariante c)* beinhaltet eine prinzipiell bekannte Methode, nach der eine Verbindung der Formel V, die in der 4-Position eine Carboxylgruppe oder ein entsprechendes Carboxylderivat enthält, zu einer erfindungsgemäßen 4-Formylverbindung der Formel I reduziert wird. Neben der Carboxylgruppe selbst kommen als geeignete Carbonsäurederivate z.B. Ester, Säurechloride, Säureanhydride, Säureamide, Säurehydrazide oder Nitrile in Betracht. Die Umwandlung der genannten funktionellen Gruppen in die Formylgruppe ist im Prinzip bekannt. (vgl. z.B. "Compendium of Organic Synthetic Methods", Verlag John Wiley u. Sons, Inc., (1971), Seiten 132 bis 137, 148 bis 150, 152 bis 153 und 166 bis 168).

Nach dem *Verfahren d)* wird eine Verbindung der Formel VI in bekannter Weise mit einem Vilsmeier-Haack Komplex wie unter Verfahren a) beschrieben umgesetzt. Die Ausgangsverbindungen VI, erhält man beispielsweise analog zu dem in J. Heterocyclic Chem. 7, (1970), Seite 615 beschriebenen Verfahren.

Nach dem *Verfahren e)* wird eine Verbindung der Formel VII mit Phosphoroxichlorid bzw. -bromid in mindestens äquivalenten Mengen, vorzugsweise mit 3—5fachen Überschuß in die erfindungsgemäßen Verbindungen überführt. Als Lösungsmittel kommen indifferente aprotische Lösungsmittel wie Benzol, Toluol, Chlorbenzol, Chloroform oder Tetrachlorkohlenstoff in Frage. Die Reaktion wird im allgemeinen zwischen 30 und 100°C, vorzugsweise zwischen 50 und 80°C ausgeführt.

Nach *Verfahren f)* ist der aromatische Rest $R_1$ einer elektrophilen Substitution zugänglich, so daß sich generell alle Substitutenten, mit denen eine solche Substitution möglich ist, in diesen Ring einführen lassen. Dazu gehören vor allem die Halogenierung oder Nitrierung, wobei der Nitrierung besonderes Interesse zukommt. Man geht dabei so vor, daß man Verbindungen der Formel I den allgemein üblichen Nitrierbedingungen unterwirft (Schwefelsäure, Salpetersäure, Eiskühlung).

Gemäß *Verfahren g)* können nun die nachträglich eingeführten oder bereits vorhandenen Substituenten $R_2$ und jene am Rest $R_1$ nachträglich verändert werden, so daß weitere Verbindungen der Formel I entstehen.

Einige Beispiele aus der Vielzahl der Möglichkeiten sollen dies erläutern. Durch Reduktion einer aromatischen Nitrogruppe erhält man eine Aminoverbindung z.B., wenn $R_1$ den 4-Nitrobenzolrest darstellt, die entsprechenden 4-Aminophenylverbindungen. Diese Reduktion wird wie üblich durchgeführt, wobei man zweckmäßigerweise mit einem Metallkatalysator wie z.B. Raney-Nickel eine Hydrierung gegebenenfalls in einem Lösungsmittel wie z.B. Äthanol durchführt. Als weiteres Beispiel wird die Alkylierung einer Aminogruppe angeführt. So läßt sich $R_1$ beispielsweise, sofern es den Aminophenylrest bedeutet, unter den üblichen Bedingungen wie z.B. mit Dimethylsulfat in die Methylamino- bzw. Dimethylaminoverbindung überführen. Die Diazotierung einer aromatischen Aminogruppe und anschließende Reaktion mit einer nukleophilen Gruppe ist eine weitere Möglichkeit zur Veränderung

bereits vorhandener Substituenten. So läßt sich beispielsweise mit salpetriger Säure (üblicherweise aus Natriumnitrit und Schwefelsäure hergestellt) ein Rest $R_1$, wenn er die 4-Amino-phenylgruppe bedeutet, bei niedrigen Temperaturen (0—5°C) in das entsprechende Diazoniumsalz überführen, das dann beispielsweise mit Salzsäure in Gegenwart von Kupferchlorid den 4-Chlorphenylrest, durch Verkochen die 4-Hydroxyphenylgruppe liefert. Die Spaltung einer Alkoxygruppe zur entsprechenden Hydroxyverbindung ist ebenfalls eine Methode zur Umwandlung der verschiedenen Substituenten. So liefert beispielsweise die Ätherspaltung einer 7-Methoxy-Verbindung ($R_2 = OCH_3$) mit beispielsweise Bromwasserstoff in wässriger Essigsäure bei Temperaturen zwischen 50°C und 120°C die entsprechende 7-Hydroxyverbindung.

Die erfindungsgemäßen Verbindungen haben wertvolle pharmakologische Eigenschaften, und zwar in erster Linie therapeutische Wirkungen auf das Zentralnervensystem. Sie hemmen durch den elektrischen Strom ausgelöste Krämpfe und zeigen auch eine durch Thiopental induzierte Narkoseverlängerung. Auf Grund dieser Eigenschaften können die erfindungsgemäßen Verbindungen als Wirkstoffe von krampfhemmenden, beziehungsweise sedierend wirkenden Arzneimitteln zur Anwendung kommen.

Die sedierende Wirkung der Isochinolin-4-aldehyde wurde an der Methamphetamin-Maus nach der von L.Ther in der Deutschen Apothekerzeitung 1953, 292 ff beschriebenen Versuchsanordnung bestimmt.

Dabei werden zwei Gruppen von jeweils drei weißen, männlichen Mäusen vom Stamm NMRI (Ivanovas) mit einem Gewicht von etwa 20 g zunächst je 0,5 mg/kg Körpergewicht einer 0,05%igen Methamphetamin-Lösung subcutan verabfolgt. 30 min. danach erhalten die Mäuse per os je 300 mg/kg Körpergewicht der in der nachfolgenden Tabelle angegebenen Substanzen, suspendiert in einer 1%igen, homogenisierten, wässrigen, Carboxymethylzellulose-Lösung. Danach werden die Mäuse in einen Glasstutzen gesetzt und nach weiteren 15 min. mit der Beobachtung begonnen.

Während der jetzt folgenden einstündigen Beobachtungszeit werden die Mäuse jede Minute einmal für eine Stunde beobachtet und es wird notiert, ob sie sich zum Zeitpunkt der Beobachtung bewegen. Gewertet wird jede Bewegung am und vom Ort. Da für jede Minute ein Punkt möglich ist, ergibt sich für die ganze Stunde eine maximal erreichbare Summe von 60 Punkten. Mäuse, die nur mit Methamphetamin behandelt wurden, erreichen diesen Wert fast regelmäßig. Setzt man den Kontrollwert eines Methamphetamin-Kollektivs gleich 100%, dann kann man die in einer Verminderung der Motilität zum Ausdruck kommende sedierende Wirkung prozentual ausdrücken. Für die erfindungsgemäßen Isochinolin-4-aldehyde ergeben sich dabei folgende Werte.

Tabell I

| Präparat | % Hemmung der motorischen Aktivität | Zahl der Mäuse |
|---|---|---|
| Kontrolle | 0 | 6 |
| Beispiel 1 | 55 | 6 |
| Beispiel 2 | 42 | 6 |
| Beispiel 3 | 49 | 6 |
| Beispiel 4 | 41 | 6 |
| Beispiel 5 | 57 | 6 |

Die neuen Verbindungen können entweder allein oder mit physiologisch verträglichen Hilfs-oder Trägerstoffen vermischt angewandt werden. Für eine orale Anwendungsform werden die aktiven Verbindungen mit den dafür üblichen Substanzen vermischt und durch übliche Methoden in geeignete Darreichungsformen gebracht, wie Tabletten, Steckkapseln, wäßrige, alkoholische oder ölige Suspensionen oder wäßrige, alkoholische oder ölige Lösungen. Als inerte Träger können z.B. Magnesiumcarbonat, Milchzucker oder Maisstärke unter Zusatz anderer Stoffe, wie z.B. Magnesiumstearat verwendet werden. Dabei kann die Zubereitung sowohl als Trocken- oder Feuchtgranulat erfolgen. Als ölige Trägerstoffe oder Lösungsmittel kommen besonders pflanzliche und tierische Öle in Betracht wie z.B. Sonnenblumenöl oder Lebertran.

Eine besondere Anwendungsform liegt in der intravenösen Applikation. Zu diesem Zweck werden die aktiven Verbindungen oder deren physiologisch verträgliche Salze mit den dafür üblichen Substanzen in Lösung gebracht. Solche physiologisch verträglichen Salze werden, z.B. mit folgenden

Säuren gebildet: Chlor-, Brom- oder Jodwasserstoffsäure, Phosphorsäure, Schwefelsäure, Methylschwefelsäure, Amidosulfonsäure, Salpetersäure, Ameisensäure, Essigsäure, Propionsäure, Bernsteinsäure, Weinsäure, Milchsäure, Malonsäure, Fumarsäure, Maleinsäure, Oxalsäure, Zitronensäure, Apfelsäure, Schleimsäure, Benzoesäure, Salicylsäure, Acetursäure, Embonsäure, Naphthalin-1,5-disulfonsäure, Ascorbinsäure, Phenylessigsäure, p-Aminosalicylsäure, Hydroxyäthansulfonsäure, Benzolsulfonsäure oder synthetische Harze, die saure Gruppen enthalten, z.B. Solche mit Ionenaustauscherwirkung. Als Lösungsmittel den entsprechenden physiologisch verträglichen Salze der aktiven Verbindungen für eine intravenöse Applikation kommen z.B. in Frage: Wasser, physiologische Kochsalzlösung oder Alkohole, wie z.B. Äthanol, Propandiol oder Glycerin, daneben auch Zuckerlösungen wie z.B. Glukose- oder Mannitlösungen, oder auch eine Mischung aus den verschiedenen genannten Lösungsmitteln.

Die Verbindungen der Formel I können gemäß der Europäischen Patentanmeldung Nr. 79 101 233.9 auch als Zwischenprodukte zur Herstellung von Isochinolinderivaten, die in 3-Stellung basisch substituiert sind, verwendet werden.

In den nachfolgenden Beispielen, werden alle Temperaturangaben in Celsiusgraden angegeben.

Beispiel 1:

*3-Chlor-1-phenyl-isochinolin-4-aldehyd*

Zu einer Lösung von 296 g 3-Chlor-4-dimethylaminomethylen-1-phenyl-isochinolin in 4 l 2n $H_2SO_4$ werden bei Raumtemperatur langsam 95 g Kaliumpermanganat in 5 g-Portionen zugegeben. Die Temperatur wird durch Kühlung unter 30°C gehalten. Man rührt bei Raumtemperatur noch 2 Stunden nach und saugt den ausgefallenen Aldehyd ab. Nach dem Trocknen und Umlösen aus Essigester isoliert man 290 g mit Schmelzpunkt 170—172°. Dies entspricht einer Ausbeute von 90%.

Das Ausgangsmaterial kann auf folgende Weise dargestellt werden:

Zu 73 ml N,N-Dimethylformamid und 400 ml Tetrahydrofuran werden 146 g Phosphoroxichlorid unter Kühlen so zugetropft, daß die Temperatur +25° nicht übersteigt. Es wird 20 Minuten bei Raumtemperatur nachgerührt. Sodann werden 56 g (0,25 Mol) 1-Phenyl-1,4-dihydro-3-(2H)-isochinolinon unter Kühlen so eingetragen, daß die Temperatur zwischen 20 und 35°C bleibt. Es wird 3 Stunden bei Raumtemperatur nachgerührt und die Reaktionslösung in 1 l 2n NaOH und 3,5 kg Eis eingetragen. Man extrahiert mehrmals mit insgesamt 1,5 l Toluol. Die Toluolphase wird mit Wasser gewaschen, getrocknet und das Lösungsmittel im Vakuum entfernt. Nach dem Verreiben des Rückstandes mit Äther können 53 g kristallines Produkt mit Schmelzpunkt 111—118° abfiltriert werden. Dies entspricht einer Ausbeute von 71 % d.Th.

Beispiel 2:

*3-Chlor-6,7-dimethoxy-1-phenyl-isochinolin-4-aldehyd*

Es wurde 6,7-Dimethoxy-1-phenyl-1,4-dihydro-3-(2H)-isochinolinon, wie im Beispiel 1, beschrieben umgesetzt. Nach dem Umkristallisieren aus Essigester erhält ma in 35 % Ausbeute ein kristallines Produkt mit Schmp. 208—210°C.

Beispiel 3:

*3-Chlor-1-(4-chlorphenyl)-6,7-dimethoxy-isochinolin-4-aldehyd*

Schmp. 263—265°

Ausb. 27 %

Beispiel 4:

*3-Chlor-1-(4-chlorphenyl)-isochinolin-4-aldehyd*

Schmp. 192—193°

Ausb. 37 %

Beispiel 5:

*3-Chlor-1-(2,4-dichlorphenyl)-isochinolin-4-aldehyd*

Schmp. 192—193°

Ausb. 32 %

Beispiel 6:

*3,6-Dichlor-1-phenyl-isochinolin-4-aldehyd*

Schmp 166—171°

Ausb. 48 %

Beispiel 7:

*3-Chlor-1-(2-methylphenyl)-isochinolin-4-aldehyd*

Schmp. 140—142°

Ausb. 37 %

Beispiel 8:

*3-Chlor-1-(3-chlorphenyl)-isochinolin-4-aldehyd*

Zu einer Lösung von 99 g N,N-Dimethylformamid in 400 ml Tetrahydrofuran werden 199 g Phosphoroxichlorid so zugetropft, daß die Temperatur 5° nicht übersteigt. Es wird 20 Minuten bei Raumtemperatur nachgerührt. Bei 0° werden dann 87,7 g 1-(3-Chlorphenyl)-1,4-dihydro-3(2H)-isochinolinon portionsweise zugegeben. Nachdem man 2 Stunden bei 0—5°C nachgerührt hat, gießt man die Reaktionslösung in eine Mischung aus 1,95 1 2n NaOH, 6 kg Eis und 1 l Toluol. Die Toluolphase wird abgetrennt, zweimal mit Wasser gewaschen und zu 1,3 l Aceton und 1,3 1 2n $H_2SO_4$ gegeben. Dieses heterogene Gemisch wird bei 20° portionsweise mit 38,8 g gut gepulvertem Kaliumpermanganat versetzt und 6 Stunden gerührt. Die Toluolphase wird abgetrennt, mit Wasser, 2n NaOH und wieder mit Wasser gewaschen, getrocknet, filtriert und das Lösungsmittel im Vakuum entfernt.

Ausb. 46 % Schmp. 169—171°C.

Beispiel 9:

*3-Chlor-1-(pyrid-4-yl)-isochinolin-4-aldehyd*

Schmp. 154—159°

Ausb. 33 %

Beispiel 10:

*3-Chlor-1-(4-fluorophenyl)-isochinolin-4-aldehyd*

Schmp. 217—218°

Ausb. 42 %

Beispiel 11:

*3-Chlor-1-phenyl-isochinolin-4-aldehyd*

Zu 2,64 g 3-Chlor-4-cyano-1-phenyl-isochinolin in 150 ml absolutem Toluol werden in einer Argonatmosphäre und bei einer Temperatur von —5° langsam 10,7 ml einer 20 % Diisobutylaluminiumhydridlösung in Toluol getropft. Man rührt 10 Minuten bei 0° und hydrolysiert mit wenig Eisessig bis zur schwach sauren Reaktion. Anschließend gibt man Wasser zu und trennt die Toluolphase ab. Die organische Phase wird mit gesättigter Natriumhydrogencarbonatlösung und dann mit Wasser gewaschen, getrocknet und das Lösungsmittel im Vakuum entfernt. Der Rückstand wird mit Äther verrieben und aus Essigester umkristallisiert.

Ausb. 85 % Schmp. 170—172°

Beispiel 12:

*3-Chloro-1-phenyl-isochinolin-4-aldehyd*

Eine Mischung von 2,69 g 3-Chlor-1-phenyl-isochinolin-4-hydroxymethylen in 50 ml Chloroform wird mit 1 g aktiviertem Mangandioxid bei Raumtemperatur für 3 Stunden gerührt. Man filtriert die Lösung und entfernt das Lösungsmittel im Vakuum. Der Rückstand schmilzt nach dem Umlösen aus Essigester bei 170—171°C und wiegt 1,4 g.

Beispiel 13:

*3-Chlor-1-(3-nitrophenyl)-isochinolin-4-aldehyd*

Bei 0°C trägt man in 30 ml konzentrierter Schwefelsäure und 1,65 ml 65 %iger Salpetersäure langsam 5,4 g 3-Chlor-1-phenyl-isochinolin-4-aldehyd ein. Die Lösung wird 2 Stdn. bei 5° gerührt und dann in 600 ml Wasser eingegossen. Der Niederschlag wird abfiltriert, gut mit Wasser gewaschen und nach dem Trocknen aus Essigester umkristallisiert. Es werden 3,4 g mit Schmp. 200—202°C isoliert.

**Patentansprüche**

1. Isochinolin-4-aldehyde der Formel I

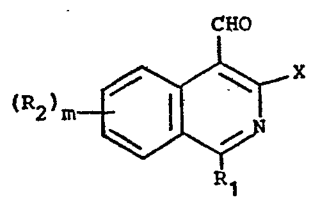

I

worin

X Brom oder Chlor bedeutet,

$R_1$ einen Phenylrest, der gegebenenfalls mit Halogen-, Hydroxy-, Nitro-, Amino- oder einer durch ein oder zwei aliphatische oder cycloaliphatische Kohlenwasserstoffreste substituierten Aminogruppe

mit zwei bis achtzehn Kohlenstoffatomen, einer Alkyl- oder Alkoxygruppe mit jeweils einem bis sechs Kohlenstoffatomen, einer Benzyloxy- oder einer Trifluormethylgruppe mono- oder disubstituiert ist, einen Pyridyl- oder Thienylrest bedeutet,

$R_2$ Wasserstoff, Halogen, Hydroxy-, Alkyl- oder Alkoxygruppe mit einem bis sechs Kohlenstoffatomen, Nitro-, Amino-, Benzyloxy, Methylendioxy- oder Äthylendioxygruppe darstellt und

m eins oder zwei darstellt,

deren physiologisch verträgliches Salze,

2. Verfahren zur herstellung von Verbindungen der Formel I gemäß Anspruch 1, dadurch gekennzeichnet, daß man

a) eine Verbindung der Formel II

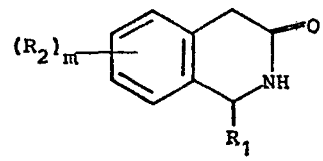

II

worin $R_1$, $R_2$ und m die zur Formel I genannte Bedeutung haben, mit einem Vilsmeier-Addukt aus einem Säureamid wie z.B. Dimethylformamid, Diäthylformamid, N,N-Methylphenylformamid oder N-Formylpiperidin und einem Säurechlorid wie z.B. Phosphoroxichlorid, Thionylchlorid, Phosgen oder Säurebromid wie Phosphoroxibromid, Phosphortribromid zu Verbindungen der Formel III umsetzt,

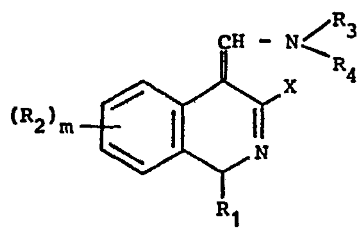

III

worin X gleich Chlor oder Brom ist und $R_3$ und $R_4$ Alkyl oder Cycloalkyl mit einem bis sechs Kohlenstoffatomen oder Phenyl bedeutet, und diese anschließend zu Verbindungen der Formel I oxydiert, oder

b) eine Verbindung der Formel IV

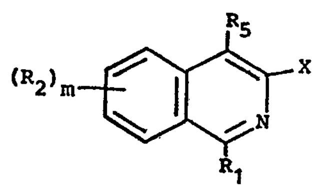

IV

worin $R_5$ einen Methyl-, Hydroxymethyl oder Aminomethylrest bedeutet, der am Stickstoffatom durch einen Alkylrest mit 1 bis 4 Kohlenstoffatomen substituiert sein kann und X, $R_1$, $R_2$ und m die oben genannte Bedeutung haben, zu einer Verbindung der Formel I oxydiert, oder

c) eine Verbindung der Formel V

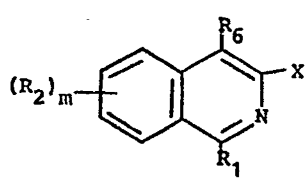

V

worin $R_6$ eine Carboxylgruppe oder ein Carbonsäurederivat bedeutet und X, $R_1$, $R_2$ und m die oben genannte Bedeutung haben, zu einer Verbindung der Formel I reduziert, oder

d) eine Verbindung der Formel VI

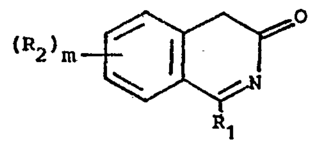

VI

worin $R_1$, $R_2$ und m die oben genannte Bedeutung haben, mit dem Vilsmeier-Haack Komplex zu einer Verbindung der Formel I umsetzt, oder

e) eine Verbindung der Formel VII

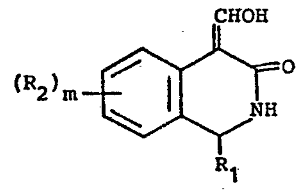

VII

worin $R_1$, $R_2$ und m die oben genannte Bedeutung haben, mit einem Säurechlorid, z.B. $POCl_3$, $SOCl_2$, zu einer Verbindung der Formel I umsetzt, oder

f) eine Verbindung der Formel I

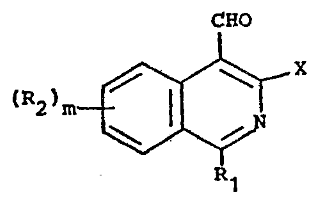

I

worin X, $R_2$ und m die zur Formel I genannte Bedeutung haben und sofern $R_1$ den Phenylring darstellt, diesen Phenylring nachträglich substituiert, oder

g) einen Rest $R_2$ oder gegebenenfalls am Phenylring in 1-Stellung vorhandene Substituenten so verändert, daß weitere Verbindungen der allgemeinen Formel I entstehen.

3. Isochinolin-4-aldehyd der Formel 1 gemäß Anspruch 1 zur Anwendung in einem therapeutischen Verfahren.

4. Pharmazeutische Zubereitung, dadurch gekennzeichnet, daß sie eine Verbindung der Formel I gemäß Anspruch 1 enthält oder aus dieser besteht.

5. Verfahren zur Herstellung einer pharmazeutischen Zubereitung nach Anspruch 4, dadurch gekennzeichnet, daß man eine Verbindung der Formel I gemäß Anspruch 1 gegebenenfalls zusammen mit üblichen pharmazeutischen Trägern und/oder Stabilisatoren in eine für pharmazeutische Zwecke geeignete Darreichungsform bringt.

**Revendications**

1. Isoquinoléine-4-carbaldéhydes de formule I

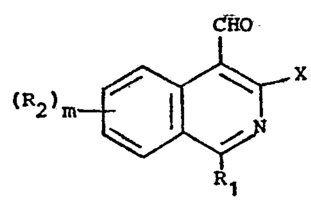

I

dans laquelle:

X représente le brome ou le chlore,

$R_1$ représente un radical phényle, qui est éventuellement mono- ou di-substitué par un halogène, un groupe hydroxy, nitro, amino ou amino substitué par un ou deux radicaux hydrocarbonés aliphatique ou cycloaliphatique ayant de 2 à 18 atomes de carbone, un groupe alkyle ou alcoxy

ayant de 1 à 6 atomes de carbone; un groupe benzyloxy ou trifluorométhyle; ou un radical pyridyle ou thiényle.

$R_2$ représente l'hydrogène, un halogène, un groupe hydroxy, alkyle ou alcoxy ayant de 1 à 6 atomes de carbone, un groupe nitro, amino, benzyloxy, méthylènedioxy ou éthylènedioxy, et m est égal à 1 ou 2,

et leurs sels physiologiquement acceptables.

2. Procédé de préparation de composés de formule I selon la revendication 1, caractérisé en ce que:

a) on fait réagir un composé de formule II

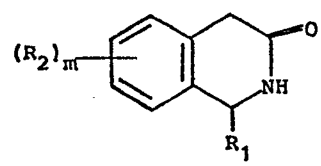

II

dans laquelle $R_1$, $R_2$ et m ont les significations indiquées pour la formule I, avec un produit d'addition de Vilsmeier obtenu à partir d'un amide d'acide tel que par exemple le diméthylformamide, le diéthylformamide, le N,N-méthylphénylformamide ou la N-formyl-pipéridine et d'un chlorure d'acide tel que par exemple l'oxychlorure de phosphore, le chlorure de thionyle, le phosgène ou d'un bromure d'acide tel que l'oxybromure de phosphore, le tribromure de phosphore, pour obtenir un composé de formule III

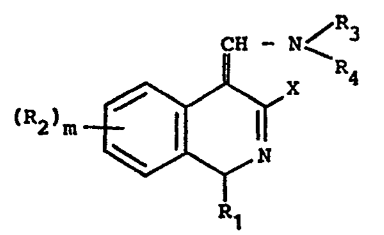

III

dans laquelle X représente le chlore ou le brome et $R_3$ et R représentent un groupe alkyle ou cycloalkyle ayant de 1 à 6 atomes de carbone ou un groupe phényle, et on oxyde ensuite ce composé en un composé de formule I, ou

b) on oxyde un composé de formule IV

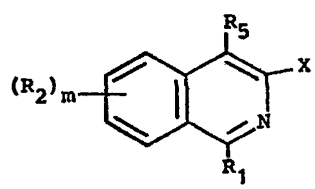

IV

dans laquelle $R_5$ représente un radical méthyle, hydroxyméthyle, ou aminométhyle, qui peut être substitué sur l'atome d'azote par un radical alkyle ayant de 1 à 4 atomes de carbone, et X, $R_1$, $R_2$ et m ont lest significations indiquées ci-dessus, en un composé de formule I, ou

c) on réduit un composé de formule V

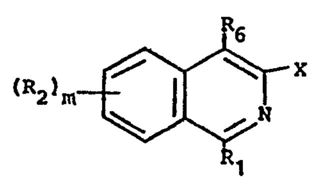

V

dans laquelle $R_6$ représente un groupe carboxyle ou un dérivé d'acide carboxylique et X, $R_1$, $R_2$ et m ont les significations indiquées ci-dessus, en un composé de formule I, ou

d) on fait réagir un composé de formule VI

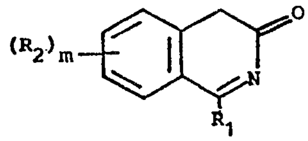

VI

dans laquelle $R_1$, $R_2$ et m ont les significations indiquées ci-dessus avec le complexe de Vilsmeier-Haack pour obtenir un composé de formule I, ou

e) on traite un composé de formule VII

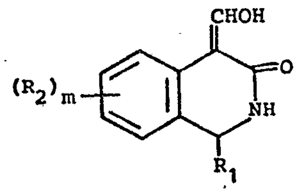

VII

dans laquelle $R_1$, $R_2$ et m ont les significations indiquées ci-dessus, par un chlorure d'acide par exemple $POCl_3$, $SOCl_2$, pour obtenir un composé de formule I, ou

f) on substitue ultérieurement le noyau phényle d'un composé de formule I

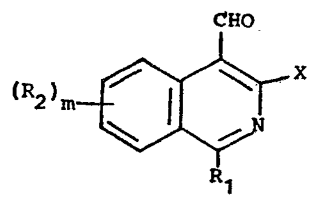

I

dans laquelle X, $R_2$ et m ont les significations indiquées ci-dessus, dans la mesure où $R_1$ représente un noyau phényle, ou

g) on modifie un radical $R_2$ ou des substituants éventuellement présents sur le noyau phényle en position 1, de façon à obtenir d'autres composées de formule générale I.

3. Utilisation des isoquinoléine-4-carbaldéhydes de formule I selon la revendication 1, dans un procédé thérapeutique.

4. Composition pharmaceutique, caractérisée en ce qu'elle contient un composé de formule I selon la revendication 1 ou consiste en ce composé.

5. Procédé de préparation d'une composition pharmaceutique selon la revendication 4, caractérisé en ce qu'on met sous une forme d'administration appropriée pour un but pharmaceutique, un composé de formule I selon la revendication 1, éventuellement en mélange avec des véhicules et/ou des stabilisants usuellement utilisés dans les produits pharmaceutiques.

## Claims

1. Isoquinoline 4-aldehydes of the formula I

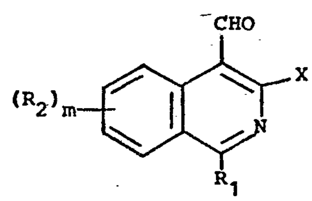

I

in which X is bromine or chlorine,

$R_1$ is phenyl or phenyl mono- or disubstituted by halogen, hydroxy, nitro, amino, or amino substituted by one or two aliphatic or cycloaliphatic hydrocarbon groups having from 2 to 18 carbon atoms, alkyl or alkoxy each having from 1 to 6 carbon atoms, benzoyloxy, or trifluoromethyl, or is pyridyl or thienyl;

$R_2$ is hydrogen, halogen, hydroxy, alkyl or alkoxy having from 1 to 6 carbon atoms, nitro, amino,

benzyloxy, methylene dioxy or ethylene dioxy and m is 1 or 2; and the physiologically tolerated salts thereof.

2. A process for the manufacture of a compound of the formula I as defined in claim 1, wherein

a) a compound of formula II

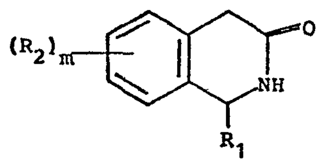

II

in which $R_1$, $R_2$ and m have the same meaning as in formula I is reacted with a Vilsmeier adduct of an acid amide, for example dimethyl formamide, diethyl formamide, N,N-methylphenyl formamide or N-formylpiperidine, and an acid chloride, for example phosphorus oxichloride, thionyl chloride, phosgene, or an acid bromide such as phosphorus oxibromide or phosphorus tribromide, to give a compound of formula III

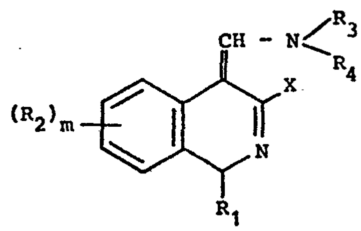

III

in which X is chlorine or bromine and $R_3$ and $R_4$ denote alkyl or cycloalkyl having from 1 to 6 carbon atoms or phenyl and the compound III obtained is then oxidized to a compound of formula I; or

b) a compound of formula IV

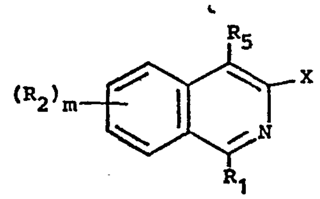

IV

in which $R_5$ denotes methyl, hydroxymethyl or aminomethyl which may be substituted at the nitrogen atom by alkyl having from 1 to 4 carbon atoms and X, $R_1$, $R_2$ and m have the aforesaid meaning is oxidized to give a compound of formula I; or

c) a compound of formula V

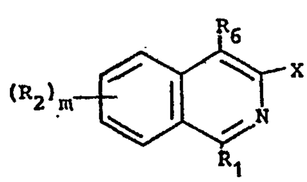

V

in which $R_6$ is a carboxy group or a derivative of a carboxy acid and X, $R_1$, $R_2$ and m have the aforesaid meaning is reduced to a compound of formula I; or

d) a compound of formula VI

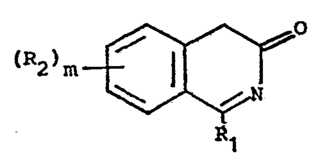

VI

in which $R_1$, $R_2$ and m have the aforesaid meaning is reacted with a Vilsmeier-Haack complex compound to give a compound of formula I; or

e) a compound of formula VII

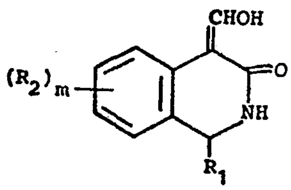

VII

in which $R_1$, $R_2$ and m have the aforesaid meaning is reacted with an acid chloride, for example $POCl_3$ or $SOCl_2$, to give a compound of formula I; or

f) in a compound of formula I

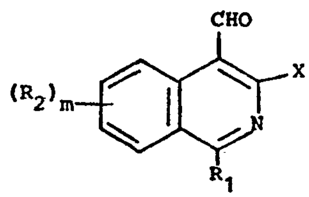

I

in which X, $R_2$ and m have the aforesaid meaning and $R_1$ denotes a phenyl ring this phenyl ring is subsequently substituted; or

g) a radical $R_2$ or a substituent in 1-position at the phenyl ring is modified to obtain another compound of formula I.

3. Isoquinoline-4-aldehyde of formula I as claimed in claim 1 for use in a therapeutical process.

4. Pharmaceutical preparation containing or consisting of a compound of formula I as claimed in claim 1.

5. Process for the manufacture of a pharmaceutical preparation as claimed in claim 4, wherein a compound of formula I as defined in claim 1 is brought into a form of administration suitable for pharmaceutical purposes, optionally together with the usual pharmaceutical carriers and/or stabilizers.